# EUROPÄISCHE PATENTSCHRIFT

(11) **EP 2 062 556 B1**
(45) Veröffentlichungstag und Bekanntmachung des Hinweises auf die Patenterteilung: **11.08.2010**
(21) Anmeldenummer: 07022726.9
(22) Anmeldetag: 23.11.2007
(51) Int. Cl.: A61F 13/551, A61F 13/84

(54) **Verpacktes Hygieneprodukt zur Aufnahme von Körperflüssigkeiten und/oder -ausscheidungen und Verfahren zur Herstellung eines derartigen Produkts**
Packaged hygiene product for absorbing bodily fluids and/or excrements and method for manufacturing such a product
Produit hygiénique emballé destiné à la réception de liquides corporels et/ou des fuites corporelles et procédé destiné à la fabrication d'un tel produit

(43) Veröffentlichungstag der Anmeldung: 27.05.2009
(73) Patentinhaber: Winkler + Dünnebier Aktiengesellschaft, 56562 Neuwied (DE)
(72) Erfinder: Wald, Ulrich, 56170 Bendorf (DE)
(74) Vertreter: Müller, Gerald Christian

(56) Entgegenhaltungen:
- EP-A- 0 841 049
- EP-A- 1 400 223
- DE-B3-102006 055 138
- US-A1- 2002 183 709

## Beschreibung

### I. Anwendungsgebiet

Die vorliegende Erfindung betrifft ein verpacktes Hygieneprodukt zur Aufnahme von Körperflüssigkeiten und/oder -ausscheidungen sowie ein Verfahren zur Herstellung eines derartigen Produktes. Unter Hygieneprodukt im Sinne der vorliegenden Erfindung sind insbesondere Slipeinlagen, Damenbinden, Windeln, Papiertaschentücher und ähnliche Produkte zu verstehen.

### II. Technischer Hintergrund

Aus der DE 102 43 156 A1 ist ein verpacktes Hygieneprodukt insbesondere in Form einer verpackten dreigefalteten Slipeinlage bekannt, bei der die so genannte Wäscheseite der Slipeinlage mit Hilfe eines Klebemittels an einer Innenseite einer Verpackungsfolie befestigt ist. Die Verpackungsfolie weist einen Durchbruch auf, durch den hindurch ein Teilbereich des an der Wäscheseite der Slipeinlage befindlichen Klebemittels zugänglich ist bzw. frei liegt. Eine Teilfläche eines über ein Ende der Slipeinlage hinausragenden Schließlappenbereichs der Verpackungsfolie kann durch den Durchbruch hindurch mit dem an der Wäscheseite befindlichen Klebemittel in Kontakt gebracht werden, so dass dadurch die Slipeinlage in einem verpackten Zustand gehalten wird. Im Rahmen der Herstellung dieser bekannten verpackten Slipeinlage ist es erforderlich, das Klebemittel auf die Wäscheseite der Slipeinlage aufzubringen. Dies hat sich aus produktionstechnischer Sicht als schwierig erwiesen.

Aus der EP 0 841 049 A1 ist ein verpacktes Hygieneprodukt insbesondere in Form einer verpackten dreigefälteten Slipeinlage bekannt, die mit Hilfe eines länglichen Klebestreifens in ihrem verpackten Zustand gehalten wird. Der Klebestreifen weist nicht über seine gesamte Fläche hinweg eine Klebewirkung auf. An einem Ende des Klebestreifens ist ein Bereich ohne Klebewirkung vorgesehen, so dass er als Aufreißlasche fungierend mit den Fingern von der Verpackungsfolie abgehoben und der gesamte Klebestreifen derart abgezogen werden kann, dass sich die Verpackung öffnet

Die im Rahmen der Herstellung derartiger verpackter Hygieneprodukte verwendeten Klebestreifen werden von Klebebändern abgeschnitten, die in auf Vorratsrollen aufgewickelter Form am Markt erhältlich sind. Die Klebebänder werden in der Herstellungsmaschine in eine geeignete Form geschnitten und die dadurch gewonnenen Klebestreifen werden an der gewünschten Stelle auf die Verpackungsfolie aufgebracht. Um die vorangehend erwähnte Aufreißlasche zu ermöglichen, weisen die am Markt erhältlichen Klebebänder im Bereich einer Seitenkante keinen Klebefilm auf, während der gesamte übrige Bereich der Klebebänder mit einem Klebefilm versehen ist.

In der Herstellungsmaschine ist das Aufbringen des von dem Klebeband abgeschnittenen Klebestreifens stets in Transportrichtung der herzustellenden Produkte durch die Herstellungsmaschine erforderlich. Darüber hinaus durchlaufen dreigefaltete Slipeinlagen die Herstellungsmaschine derart, dass die Schließkante der Verpackungsfolie quer zu der Transportrichtung verläuft. Aus diesen Gründen sowie aufgrund der voranstehend erwähnten Klebefilmverteilung an den am Markt erhältlichen Klebebändern ist es bei dem aus der EP 0 841 049 A1 bekannten Hygieneprodukt erforderlich, dasselbe vor dem Aufbringen des Klebestreifens um 90° um seine Hochachse zu drehen. Dies ist naturgemäß mit einem an sich unerwünschten produktionstechnischen Aufwand verbunden.

### III. Darstellung der Erfindung

### a) Technische Aufgabe

Es ist daher die Aufgabe der vorliegenden Erfindung, ein verpacktes Hygieneprodukt zur Aufnahme von Körperflüssigkeiten und/oder -ausscheidungen sowie ein Verfahren zur Herstellung eines derartigen Produktes zu schaffen, bei dem einerseits ein unmittelbares Aufbringen eines Klebemittels auf die Wäscheseite des Hygieneprodukts nicht erforderlich ist und andererseits als Verschluss dienende Klebestreifen, die aus am Markt erhältlichen Klebebändern zurechtgeschnitten werden, ohne die Notwendigkeit einer Drehung des Hygieneprodukts um seine Hochachse auf die jeweilige Verpackungsfolie aufgebracht werden können.

### b) Lösung der Aufgabe

Diese Aufgabe wird mit einem verpackten Hygieneprodukt bzw. mit einem Verfahren zur Herstellung eines verpackten Hygieneprodukts mit den Merkmalen des Anspruchs 1 bzw. 7 gelöst. Weitere Ausgestaltungen der vorliegenden Erfindung ergeben sich aus den Unteransprüchen.

Erfindungsgemäß wird vorgeschlagen, in dem Ober ein Ende des Hygieneprodukts hinausragenden Schließlappenbereich des Verpackungsmaterials wenigstens einen Durchbruch anzuordnen, durch den hindurch ein mit einem Klebefilm versehener Klebestreifen an der Außenseite des Verpackungsmaterials befestigt bzw. befestigbar ist, um das Hygieneprodukt in verpacktem Zustand zu halten.

Das erfindungsgemäß verpackte Hygieneprodukt bringt unter anderem den Vorteil mit sich, dass ein zur Befestigung des Hygieneprodukts an dem Verpackungsmaterial erforderliches Klebemittel im Rahmen der Produktherstellung direkt auf das Verpackungsmaterial selbst und nicht auf die Wäscheseite des Hygieneprodukts aufgetragen werden kann. Das vorzugsweise in Folienform zur Verfügung gestellte Verpackungsmaterial kann von einer Vorratsrolle abgewickelt und anschließend auf einfache Weise beleimt werden. Darüber hinaus ragt der Klebestreifen erfindungsgemäß nicht wie bei dem aus der EP 0 841 049 A1 bekannten Hygieneprodukt Ober die den Schließlappenbereich abschließende Kante hinaus, so dass das erfindungsgemäße Hygieneprodukt auf ein kleineres Format zusammengelegt bzw. gefaltet werden kann.

Vorzugsweise weist die den Schließlappenbereich abschließende Kante eine über sie hervorstehende Grifflasche auf. Durch Ziehen an der Grifflasche wird das verpackte Hygieneprodukt geöffnet, wobei sich der durch den Durchbruch hindurch mit der Außenseite des Verpackungsmaterials in Klebeverbindung befindliche Klebefilm von der Außenseite löst. Da der Klebefilm eine Fläche aufweist, die vorzugsweise größer als die Fläche des Durchbruchs in dem Schließlappenbereich ist, bleibt der Klebestreifen auch bei geöffnetem Produkt vorzugsweise an dem Schließlappenbereich haften. Nach dem Öffnen des verpackten Hygieneprodukts kann letzteres in üblicher Weise von dem Verpackungsmaterial zerstörungsfrei entfernt und gebraucht werden.

Erfindungsgemäß kann das Verpackungsmaterial in vorteilhafter Weise zur Entsorgung des gebrauchten Hygieneprodukts verwendet werden. Hierzu weist der noch an dem Schließlappenbereich des Verpackungsmaterials haftende Klebestreifen wenigstens eine Abziehlasche in Form eines Nichtklebebereichs ohne Klebefilm auf. Vorzugsweise nach dem Einpacken des gebrauchten Hygieneprodukts in das während der Gebrauchsdauer aufbewahrte Verpackungsmaterial kann der Klebestreifen von dem Schließlappenbereich abgezogen und zur Herstellung eines Klebeverschlusses der in der EP 0 841 049 A1 gezeigten Art verwendet werden. Ein an sich unerwünschtes Nachlassen der Klebewirkung des Klebestreifens kann nur durch Verschmutzung derjenigen Klebefilmfläche eintreten, die in geöffnetem Zustand der Verpackung durch den Durchbruch hindurch freiliegt. Erfindungsgemäß wird somit ein sicheres Wiederverschließen des Verpackungsmaterials nach dem Einpacken des gebrauchten Hygieneprodukts gewährleistet.

Aus produktionstechnischer Sicht weist die vorliegende Erfindung weiterhin den Vorteil auf, dass von handelsüblichen Klebebändern abgeschnittene Klebestreifen in derjenigen Richtung auf den Schließlappenbereich aufgebracht werden können, in welcher die Hygieneprodukte durch die Herstellungsmaschine transportiert werden. Eine Gierbewegung der Hygieneprodukte in Form einer 90°-Drehung um ihre Hochachse erübrigt sich erfindungsgemäß. Dies liegt daran, dass die Abziehrichtung zum Abziehen des Klebestreifens zum Zwecke des Wiederverschließens des Verpackungsmaterials im Rahmen der Entsorgung des gebrauchten Hygieneprodukts quer zu der Transportrichtung der Hygieneprodukte durch die Herstellungsmaschine verläuft.

Im Rahmen des erfindungsgemäßen Verfahrens zur Herstellung des verpackten Hygieneprodukts kann letzteres zusammengelegt oder zusammengerollt werden, wobei vorzugsweise ein Zusammenfalten erfolgt. Das Herstellungsverfahren ermöglicht ein Aufbringen des Klebestreifens auf den Schließlappenbereich des Verpackungsmaterials zeitlich vor oder nach dem Zusammenfalten des Hygieneprodukts samt Verpackungsmaterial. Besonders vorteilhaft ist das Aufbringen des Klebestreifens vor dem Hersteltungsschritt des Zusammenfaltens, da in diesem Fall ein sofortiges Verschließen des Verpackungsmaterials am Ende des Faltvorgangs möglich ist.

In diesem Zusammenhang wird ausdrücklich auf Fig. 8 der DE 101 27 278 A1 Bezug genommen, die beispielhaft verschiedene Herstellungsschritte zur Herstellung nicht erfindungsgemäßer, verpackter Hygieneprodukte zeigt. Ein Teil derjenigen Modifikationsmaßnahmen, die bei diesem bekannten Herstellungsverfahren zur Umrüstung auf das erfindungsgemäße Herstellungsverfahren notwendig sind, besteht darin, die Klebestreifen entweder in Fig. 8 oberhalb der Klebstoff-Auftrage-Einrichtung 18 auf die Folienbahn 15 oder in Fig. 8 rechts neben der Faltvorrichtung 29 auf das fertige verpackte Hygieneprodukt 2 aufzubringen.

### c) Ausführungsbeispiel

Nachfolgend wir eine Ausführungsform der vorliegenden Erfindung beispielhaft anhand einer dreigefalteten Slipeinlage mit erfindungsgemäß angeordnetem Klebestreifen beschrieben. Es zeigen:
- Fig. 1:: Eine Aufsicht auf eine Ausführungsform der erfindungsgemäßen verpackten Slipeinlage; und
- Fig. 2:: eine Schnittansicht gemäß Schnitt A-A in Fig. 1.

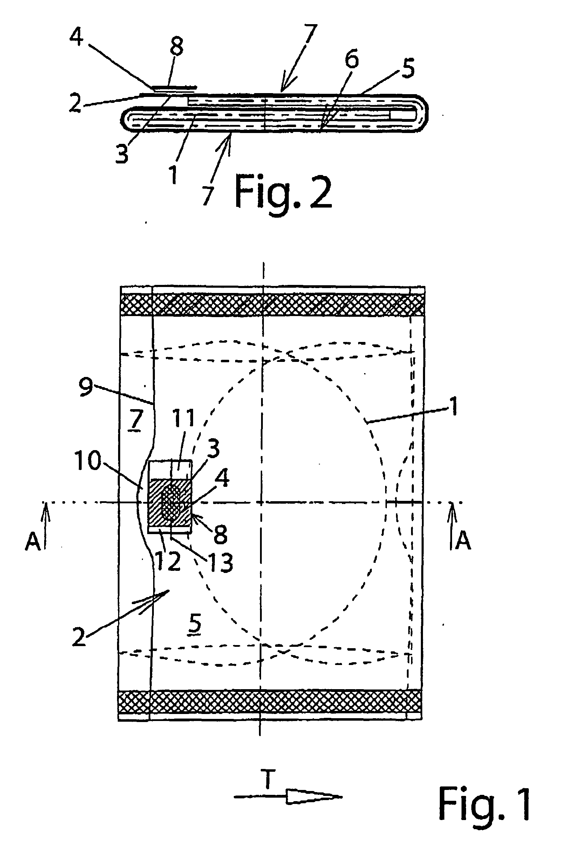

Fig. 1 zeigt eine Aufsicht auf eine Ausführungsform einer erfindungsgemäßen verpackten Slipeinlage 1. Die Umrisse der dreigefalteten Slipeinlage 1 sind in Fig. 1 in gestrichelten Linien dargestellt. Die Slipeinlage 1 weist eine bei ihrem Gebrauch der Wäsche der Benutzerin zugewandte Wäscheseite auf, die in an sich bekannter Weise lösbar an der Innenseite 6 einer Verpackungsfolie 5 befestigt ist. Die Dreifaltung (zwei Faltvorgänge, drei Faltlagen) der Slipeinlage 1 erfolgt ebenso in an sich bekannter Weise, beispielsweise derart, wie es in der DE 102 43 156 A1 (siehe dort insbesondere Fig. 5A, 5B und 5C sowie zugehörige Beschreibung) beschrieben ist. Bei der Außenseite 7 handelt es sich um diejenige Seite der Verpackungsfolie 5, die der Innenseite 6 bzw. der Slipeinlage 1 abgewandt ist.

In den Fig. 1 und 2 ist ein Schließlappenbereich 2 zu erkennen, der in den Figuren nach links Ober ein Ende der Slipeinlage 1 hinausragt. Er bildet einen Teil bzw. eine Zone der Verpackungsfolie 5. In dem Schließlappenbereich 2 ist ein Durchbruch 3 vorgesehen, der im Wesentlichen eine längliche Ovalform aufweist. Durch den Durchbruch 3 hindurch ist ein Flächenbereich der Außenseite 7 der Verpackungsfolie 5 zugänglich, der seiner Größe nach der Fläche des Durchbruchs 3 entspricht.

Auf der Außenseite 7 des Schließlappenbereichs 2 ist ein Klebestreifen 8 derart angeordnet, dass er den Durchbruch 3 vollständig überdeckt. Der Klebestreifen 8 weist bei der gezeigten Ausführungsform eine Rechteckform auf. An seiner in Fig. 2 unteren Seite ist der Klebestreifen 8 mit einem Klebefilm 4 versehen, der nicht die gesamte Fläche des Klebestreifens 8 bedeckt. Durch den Durchbruch 3 hindurch steht ein Teil des Klebefilms 4 mit einem verdeckt unter dem Schließlappenbereich 2 befindlichen Flächenbereich der Außenseite 7 der Verpackungsfolie 5 in Kontakt, so dass dadurch der Schließlappenbereich 2 verschlusssicher an dem entsprechenden Flächenbereich der Außenseite 7 der Verpackungsfolie 5 festgehalten wird.

An seinem in Fig. 1 oberen Ende weist der Klebestreifen 8 einen als Abziehlasche fungierenden Nichtklebebereich 11 auf, in welchem sich an der Unterseite des Klebestreifens 8 kein Klebefilm befindet. Bei der gezeigten Ausführungsform ist auch an dem in Fig. 1 unteren Ende des Klebestreifens 8 ein Nichtklebebereich 12 ohne Klebefilm angeordnet, der eine flächenmäßig geringere Erstreckung als der Nichtklebebereich 11 aufweist. Die in Fig. 1 obere Kante des Nichtklebebereichs 11 und die In Fig. 1 untere Kante des Nichtklebebereichs 12, d.h. die obere und untere Kante des Klebestreifens 8, stellen einen Teilabschnitt der Seitenränder der am Markt erhältlichen, auf Vorratsrollen aufgewickelten Klebebänder dar, von denen der Klebestreifen 8 abgeschnitten wird.

Wie in Fig. 1 zu erkennen ist, wird der Schließlappenbereich 2 der Verpackungsfolie 5 nach links von einer Kante 9 abgeschlossen. Bei der Kante 9 handelt es sich um eine der beiden kurzen Rechteckseiten der entfalteten Verpackungsfolie 5. Sie weist eine Grifflasche 10 in Form einer in Fig. 1 nach links weisenden Auswölbung auf. Die Längsachse 13 sowohl des Durchbruchs 3 als auch des Klebestreifens 8 verläuft im Wesentlichen parallel zu der den Schließlappenbereich 2 abschließenden Kante 9.

Zum Öffnen der verpackten Slipeinlage 1 wird an der Grifflasche 10 gezogen. Dabei löst sich der größenmäßig der Fläche des Durchbruchs 3 entsprechende Bereich des Klebefilms 4 von der Außenseite 7 der Verpackungsfolie 5. Die Slipeinlage 1 kann entnommen und benutzt werden.

Soll die benutzte Slipeinlage 1 in der Verpackungsfolie 5 entsorgt werden, so kann der Klebestreifen 8 durch Ziehen an seinem Nichtklebebereich 11 von dem Schließlappenbereich 2 abgezogen und - nach dem Einpacken der gebrauchten Slipeinlage 1 in der Verpackungsfolie 5 - zum Wiederverschließen derart über die Kante 9 geklebt werden, dass ein Teil des Klebefilms 4 an der Außenseite 7 und ein anderer Teil des Klebefilms 4 an dem Schließlappenbereich 2 haftet. Auf diese Weise ist die gebrauchte Slipeinlage 1 sicher verschlossen. Bei dieser Art des Wiederverschließens steht wenigstens derjenige Bereich des Klebefilms 4 als wirksames Haftmittel zur Verfügung, der sich zuvor außerhalb der Fläche des Durchbruchs 3 befunden und den Klebestreifen 8 während der Aufbewahrungszeit der Verpackungsfolie 5 an dem Schließlappenbereich 2 gehalten hat. Der etwaige Verlust der Haftwirkung des Bereichs des Klebefilms innerhalb des Durchbruchs 3 durch Verschmutzung oder ähnliches kann erfindungsgemäß daher in Kauf genommen werden.

Um ein zerstörungsfreies Abziehen des Klebestreifens 8 von dem Schließlappenbereich 2 zu erleichtern, kann letzterer zumindest in demjenigen Flächenbereich um den Durchbruch 3 herum, in welchem bei aufgeklebtem Klebestreifen 8 der Klebefilm 4 zu liegen kommt, mit einer Silikonbeschichtung versehen werden.

Wie in Fig. 1 zu erkennen ist, besteht bei der ungebrauchten bzw. originalverpackten Slipeinlage 1 keine Notwendigkeit, den Klebestreifen 8 über die Kante 9 hinweg an der Außenseite 7 der Verpackungsfolie 5 zu befestigen. Bei Bedarf könnte daher die Kante 9 in Fig. 1 noch weiter nach links verlagert werden, d.h. das Horizontalmaß der in Fig. 1 gezeigten verpackten Slipeinlage 1 könnte auf eine geringere Abmessung gefaltet werden.

In Fig. 1 ist mit dem nach rechts weisenden Pfeil T die Transportrichtung gekennzeichnet, in welcher die Slipeinlagen 1 samt Verpackungsfolie 5 während des Produktionsprozesses durch die Herstellungsmaschine transportiert werden (vergleiche hierzu Fig. 8 der DE 101 27 278 A1). Die auf der Längsachse 13 des Klebestreifens 8 senkrecht stehende Achse desselben verläuft parallel zu der Transportrichtung T und entspricht der Längsrichtung der am Markt erhältlichen, auf Vorratsrollen aufgewickelten Klebebänder, so dass die während des Herstellungsprozesses von den Klebebändern abgeschnittenen Klebestreifen 8 jeweils in Transportrichtung T auf den Schließlappenbereich 2 geklebt werden können. Die verpackte Slipeinlage 1 muss hierzu nicht um ihre Hochachse gedreht werden.

### BEZUGSZEICHENLISTE

- 1: Slipeinlage
- 2: Schließlappenbereich
- 3: Durchbruch
- 4: Klebefilm
- 5: Verpackungsmaterial
- 6: Innenseite
- 7: Außenseite
- 8: Klebestreifen
- 9: Kante des Schließlappenbereichs
- 10: Grifflasche
- 11,12: Nichtklebebereich
- 13: Längsachse

- T: Transportrichtung

## Patentansprüche

1. Verpacktes Hygieneprodukt zur Aufnahme von Körperflüssigkeiten und/oder
- ausscheidungen, umfassend
• das Hygieneprodukt (1), welches eine dem Körper zugewandte Körperseite sowie eine von dem Körper abgewandte Wäscheseite aufweist und zum Verpacken zusammenleg- oder -rollbar ist, und
• ein Verpackungsmaterial (5), welches eine dem Hygieneprodukt (1) zugewandte Innenseite (6) und eine von dem Hygieneprodukt (1) abgewandte Außenseite (7) aufweist, wobei die Wäscheseite des Hygieneprodukts (1) an der Innenseite (6) befestigt ist und das Verpackungsmaterial (5) mit einem Schließlappenbereich (2) über ein Ende des Hygieneprodukts (1) hinausragt,
**dadurch gekennzeichnet, dass**
in dem Schließlappenbereich (2) wenigstens ein Durchbruch (3) angeordnet ist, durch den hindurch ein mit einem Klebefilm (4) versehener Klebestreifen (8) an der Außenseite (7) des Verpackungsmaterials (5) befestigt ist, um das Hygieneprodukt (1) in verpacktem Zustand zu halten.

2. Verpacktes Hygieneprodukt nach Anspruch 1,
**dadurch gekennzeichnet, dass**
der Klebefilm (4) eine Fläche aufweist, die größer als die Fläche des Durchbruchs (3) ist.

3. Verpacktes Hygieneprodukt nach Anspruch 1 oder 2,
**dadurch gekennzeichnet, dass**
der Schließlappenbereich (2) eine Kante (9) mit einer hervorstehenden Grifflasche (10) aufweist.

4. Verpacktes Hygieneprodukt nach einem der vorangehenden Ansprüche, **dadurch gekennzeichnet, dass**
der Klebestreifen (8) wenigstens eine Abziehlasche zum Abziehen von dem Verpackungsmaterial (5) in Form eines Nichtklebebereichs (11) ohne Klebefilm aufweist.

5. Verpacktes Hygieneprodukt nach einem der vorangehenden Ansprüche, **dadurch gekennzeichnet, dass**
der Schließlappenbereich (2) zumindest in demjenigen Flächenbereich um den Durchbruch (3) herum, in welchem bei aufgeklebtem Klebestreifen (8) der Klebefilm (4) zu liegen kommt, eine Silikonbeschichtung aufweist.

6. Verpacktes Hygieneprodukt nach einem der vorangehenden Ansprüche, **dadurch gekennzeichnet, dass**
der Klebestreifen (8) und/oder der Durchbruch (3) eine längliche Form aufweisen, deren/dessen Längsachse (13) im Wesentlichen parallel zu der den Schließlappenbereich (2) abschließenden Kante (9) verlauft.

7. Verfahren zur Herstellung eines verpackten Hygieneprodukts (1) zur Aufnahme von Körperflüssigkeiten und/oder -ausscheidungen, umfassend die folgenden Schritte:
■ Bereitstellen des Hygieneprodukts (1) mit einer dem Körper zugewandten Körperseite sowie einer von dem Körper abgewandten Wäscheseite, und
■ Bereitstellen eines Verpackungsmaterials (5) mit einer Innenseite (6) und einer Außenseite (7),
■ wobei die Wäscheseite des Hygieneprodukts (1) an der Innenseite (6) des Verpackungsmaterials (5) derart befestigt wird, dass ein Schließlappenbereich (2) des Verpackungsmaterials (5) über ein Ende des Hygieneprodukts (1) hinausragt, und das Hygieneprodukt (1) zusammen mit dem Verpackungsmaterial (5) zusammengelegt oder -gerollt wird,
**dadurch gekennzeichnet, dass**
in den Schließlappenbereich (2) wenigstens ein Durchbruch (3) eingebracht wird und durch den Durchbruch (3) hindurch ein mit einem Klebefilm (4) versehener Klebestreifen (8) an der Außenseite (7) des Verpackungsmaterials (5) befestigt wird, um das Hygieneprodukt (1) in verpacktem Zustand zu halten.

8. Verfahren nach Anspruch 7,
**dadurch gekennzeichnet, dass**
der Klebestreifen (8) mit einem Klebefilm (4), der eine Fläche aufweist, die größer als die Fläche des Durchbruchs (3) ist, vor dem Zusammenlegen oder Zusammenrollen des mit dem Verpackungsmaterial (5) verbundenen Hygieneprodukts (1) an dem Schließlappenbereich (2) angebracht wird.

9. Verfahren nach Anspruch 7,
**dadurch gekennzeichnet, dass**
der Klebestreifen (8) mit einem Klebefilm (4), der eine Fläche aufweist, die größer als die Fläche des Durchbruchs (3) ist, nach dem Zusammenlegen oder Zusammenrollen des mit dem Verpackungsmaterial (5) verbundenen Hygieneprodukts (1) an dem Schließlappenbereich (2) angebracht wird.

## Claims

1. Packaged hygiene product for absorbing body fluids and/or secretions, comprising
• the hygiene product (1), which has a body side facing the body and a panty side facing away from the body and can be folded or rolled for packaging purposes,
• a packaging material (5), which has an internal face (6) facing the hygiene product (1) and an external face (7) facing away from the hygiene product (1), and the panty side of the hygiene product (1) is secured to the internal face (6) and the packaging material (5) extends beyond one end of the hygiene product (1) by means of a closing flap region (2),
**characterised in that**
at least one cut-out (3) is provided in the closing flap region (2), through which an adhesive strip (8) provided with an adhesive film (4) is secured to the external face (7) of the packaging material (5) in order to retain the hygiene product (1) in the packaged state.

2. Packaged hygiene product as claimed in claim 1, **characterised in that**
the adhesive film (4) has a surface area which is bigger than the surface area of the cut-out (3).

3. Packaged hygiene product as claimed in claim 1 or 2,
**characterised in that**
the closing flap region (2) has an edge (9) with a protruding gripping tab (10).

4. Packaged hygiene product as claimed in one of the preceding claims,
**characterised in that**
the adhesive strip (8) has at least one tear-off tab intended to be torn off the packaging material (5) in the form of a non-adhesive region (11) without adhesive film.

5. Packaged hygiene product as claimed in one of the preceding claims,
**characterised in that**
the closing flap region (2) has a silicone coating at least in the surface region around the cut-out (3) in which the adhesive film (4) is positioned when the adhesive strip (8) is adhered.

6. Packaged hygiene product as claimed in one of the preceding claims,
**characterised in that**
the adhesive strip (8) and/or the cut-out (3) is of an elongate shape, the longitudinal axis (13) of which extends essentially parallel with the edge (9) terminating the closing flap region (2).

7. Method of producing a packaged hygiene product (1) for absorbing body fluids and/or secretions, comprising the following steps:
• preparing the hygiene product (1) with a body side facing the body and a panty side facing away from the body, and
• preparing a packaging material (5) with an internal face (6) and an external face (7),
• and the panty side of the hygiene product (1) is secured to the internal face (6) of the packaging material (5) so that a closing flap region (2) of the packaging material (5) extends beyond one end of the hygiene product (1), and the hygiene product (1) is folded or rolled together with the packaging material (5), **characterised in that**
at least one cut-out (3) is made in the closing flap region (2) and an adhesive strip (8) provided with an adhesive film (4) is secured to the external face (7) of the packaging material (5) through the cut-out (3) in order to retain the hygiene product (1) in the packaged state.

8. Method as claimed in claim 7,
**characterised in that**
the adhesive strip (8) with an adhesive film (4), which has a surface area that is bigger than the surface area of the cut-out (3), is attached to the closing flap region (2) prior to folding or rolling the hygiene product (1) joined to the packaging material (5).

9. Method as claimed in claim 7,
**characterised in that**
the adhesive strip (8) with an adhesive film (4), which has a surface area that is bigger than the surface area of the cut-out (3), is attached to the closing flap region (2) after folding or rolling the hygiene product (1) joined to the packaging material (5).

## Revendications

1. Produit hygiénique emballé destiné à la réception de liquides corporels et/ou de fuites corporelles, englobant
• Le produit hygiénique (1), lequel présente un côté corps orienté vers le corps et un côté linge opposé au corps et lequel peut être plié ou enroulé pour être emballé, et
• Un matériel d'emballage (5), lequel présente une face interne (6) orientée vers le produit hygiénique (1) et une face externe (7) opposée au produit hygiénique (1), sachant que le côté linge du produit hygiénique (1) est fixé à la face interne (6) et que le matériel d'emballage (5) dépasse d'une extrémité du produit hygiénique (1) avec un partie de fermeture (2),
**caractérisé en ce que**
dans la partie de fermeture (2), il est prévu au moins une perforation (3) à travers laquelle passe une bande adhésive (8) pourvue d'un film adhésif (4) qui vient se fixer sur la face externe (7) du matériel d'emballage (5) afin de maintenir le produit hygiénique (1) emballé.

2. Produit hygiénique emballé selon la revendication 1, **caractérisé en ce que**
le film adhésif (4) présente une surface qui est plus grande que la surface de la perforation (3).

3. Produit hygiénique emballé selon la revendication 1 ou 2, **caractérisé en ce que**
la partie de fermeture (2) présente un bord (9) avec une languette de saisie saillante (10).

4. Produit hygiénique emballé selon l'une des revendications précédentes, **caractérisé en ce que**
la bande adhésive (8) présente au moins une languette amovible servant à découvrir le matériel d'emballage (5) sous forme de partie non adhésive (11) sans film adhésif.

5. Produit hygiénique emballé selon l'une des revendications précédentes, **caractérisé en ce que**
La partie de fermeture (2) présente une couche de silicone au moins sur la partie entourant la perforation (3), à laquelle le film adhésif (4) adhère lorsque la bande adhésive (8) est collée.

6. Produit hygiénique emballé selon l'une des revendications précédentes, **caractérisé en ce que**
la bande adhésive (8) et/ou la perforation (3) présentent une forme allongée, dont l'axe longitudinal (13) est globalement parallèle au bord (9) délimitant la partie de fermeture (2).

7. Procédé de fabrication d'un produit hygiénique emballé (1) destiné à la réception de liquides corporels et/ou de fuites corporelles, englobant les étapes suivantes :
• Préparation du produit hygiénique (1) avec un côté corps orienté vers le corps et un côté linge opposé au corps, et
• Préparation d'un matériel d'emballage (5) avec une face interne (6) et une face externe (7),
• Sachant que le côté linge du produit hygiénique (1) est fixé sur la face interne (6) du matériel d'emballage (5) de telle sorte qu'une partie de fermeture (2) du matériel d'emballage (5) dépasse d'une extrémité du produit hygiénique (1) et que le produit hygiénique (1) est plié ou enroulé avec le matériel d'emballage (5),
**caractérisé en ce que**,
dans la partie de fermeture (2), au moins une perforation (3) est effectuée et qu'à travers la perforation (3) passe une bande adhésive (8) pourvue d'un film adhésif (4) qui vient se coller sur la face externe (7) du matériel d'emballage (5), afin de maintenir le produit hygiénique (1) emballé.

8. Procédé selon la revendication 7, **caractérisé en ce que** la bande adhésive (8) avec le film adhésif (4) dont la surface est plus grande que la surface de la perforation (3) est appliquée sur la partie de fermeture (2) avant que le produit hygiénique (1) rattaché au matériel d'emballage (5) ne soit plié ou enroulé.

9. Procédé selon la revendication 7, **caractérisé en ce que** la bande adhésive (8) avec un film adhésif (4) dont la surface est plus grande que la surface de la perforation (3) est appliquée sur la partie de fermeture (2) après que le produit hygiénique (1) rattaché au matériel d'emballage (5) ait été plié ou enroulé.
